## Europäisches Patentamt
## European Patent Office
## Office européen des brevets

(19)

(11) Publication number: **0 112 299**
**A2**

(12) **EUROPEAN PATENT APPLICATION**

(21) Application number: **83830118.2**

(22) Date of filing: **10.06.83**

(51) Int. Cl.³: **C 12 N 9/02**

(30) Priority: **14.12.82 IT 4967082**

(43) Date of publication of application:
**27.06.84 Bulletin 84/26**

(84) Designated Contracting States:
**AT BE CH DE FR GB LI LU NL SE**

(71) Applicant: **Bianchi, Ines**
**Via XXIV Maggio 47**
**Grottaferrata Roma(IT)**

(72) Inventor: **Bianchi, Ines**
**Via XXIV Maggio 47**
**Grottaferrata Roma(IT)**

(74) Representative: **Fiammenghi-Domenighetti,**
**Delfina et al,**
**Racheli-Fiammenghi-Fiammenghi Via Quattro Fontane**
**31**
**I-00184 Roma(IT)**

(54) **Method for superoxidic-dismutasis purification via chromatography by affinity.**

(57) Method for obtaining purified superoxidic-dismutasis from animal tissues, in particular from erythrocytes or from hepatic tissue, through affinity chromatography or immunoabsorbent with specific purified antibodies for the enzyme.

EP 0 112 299 A2

Croydon Printing Company Ltd.

METHOD FOR SUPEROXIDIC-DISMUTASIS PURIFICATION VIA
CHROMATOGRAPHY BY AFFINITY

This invention refers to a method for purifying
superoxidic-dismutasis. More specifically, this
invention refers to a method for obtaining purified
superoxidic-dismutasis from animal tissues in particular
from the hepatic tissue or erythrocytes of the superior
animals, as the Mammalia, Primates excepted, through
chromatography by affinity on an immunoabsorbent with
specific antibodies for the enzyme.

It is well-known from prior art that several organs or
tissues of living organisms contain an enzyme which
catalyzes the dismutation reaction of the anion
superoxide, which is one of the products which develop
physiologically from the reduction of oxygen.

This enzyme, found in superior animals, contains copper,
an element which is essential for the catalytic
activity.

In view of the beneficial effects that the administration
of superoxidic-dismutasis causes in men and animals in
some pathological conditions, it is obvious that the
problem of purification and isolation of this enzyme has
gained increasing importance.

As it is well-known, superoxidic-dismutasis can be found
mainly in the liver and in the blood of superior
animals, but in rather small amounts, i.e. in the order of 0.05 - 0.1 grams per kilo of fresh tissue.

So far several methods have been proposed for the purification of superoxidic-dismutasis from hepatic tissue and erythrocytes.

These methods are generally based on the preferential removal of foreign proteins obtained through a treatment with organic solvents or proteolytic enzymes or acidic buffers. The superoxidic-dismutasis is then purified by using conventional methods for the purification of enzymes.

However, these techniques are not free from drawbacks which concern both the number and the complexity of operations needed as well as their high cost.

Therefore, the purpose of this invention is to provide a method for the purification of superoxidic-dismutasis free from the above-mentioned practical and economical drawbacks.

To this end, the method which is disclosed in this invention, is based on a selective interaction between the specific antibody, immunized suitably, and the enzyme which is present in the organ extract.

It was found out in fact that the interaction between superoxidic-dismutasis and specific antibodies, suitably selected and covalently bound on an insoluble matrix can be used for removing the tissue components which are foreign to the enzyme with a single operation. The enzyme can then be extracted without being damaged with the usual conventional tecniques. Thus the process for

purifying superoxidic-dismutasis according to this invention turns out to be more advantageous than other well-known processes thanks to its semplicity.

Therefore, the specific object of this invention is to provide a method for the purification of superoxidic-dismutasis from animal tissues and is characterized by the use of specific antibodies for the enzyme.

This invention will be thereinafter described in detail in the following example.

Example

This example concerns the purification of superoxidic-dismutasis, which is obtained from cattle erythrocytes. The preparation described is referred to one liter of cattle erythrocytes as starting material.

First of all has to be prepared the antiserum anti-superoxidic-dismutasis. For such a purpose rabbits are used, each of which is immunized through the following procedure:

a) a first inoculation of a mixture 1:1 of antigen solution (1 mg/ml) and a complete Freund adjuvant is carried out. 1 ml of this mixture is inoculated into the paw-pads of the hind legs of each rabbit;

b) after 30 days a second inoculation is carried out with the same antigenic mixture in an incomplete Freund adjuvant and in the same way as the first one . 15 days after the second inoculation has been carried out, the

0112299

— 4 —

rabbits are killed and the serum is collected.
The specific anti bodies are then purified by means of a
chromatography by affinity, according to the following
procedure: a) superoxidic-dismutasis is made insoluble
on an inert matrix. The matrix, which in this case,
consists of Sepharose 4B (Trade Mark) resin produced by
Pharmacia, is washed three times in a solution of sodium
chloride 0.15 M buffered at pH 7.2 with phosphate 15 mM
(from now on called PBS).

The resin is then resuspended at 50% in water and CNBr
dissolved in water is added to it under agitation,
keeping the pH constant at 11, by adding NaOH 1N.
After about 10 minutes the resin is washed with cold
distilled water and sodium bicarbonate 0.1 M, pH 8.6 and
filtered through a paper filter, which is placed in a
china funnel. The matrix is then brought into contact
with an equal volume of solution of superoxidic-
-dismutasis at a concentration of 3 mg/ml in sodium
bicarbonate 0.1 M, pH 8.6 and is left to react for 24
hours at + 4°C, under agitation. Washing is then carried
out by using sodium bicarbonate 0.1M, pH 8.6, acetic
acid 0.1M and PBS. Two volumes of $MgCl_2$ 3.5 M are then
passed through in order to remove the residual protein
which is non-covalently bound. Under these conditions
50%, of the enzyme binds itself to the fixed phase;
b) the specific antibodies are isolated by mixing the
rabbit's serum with the immunoabsorbent under saturant
conditions. After being washed in PBS, and until
absorption at 280 nm becomes negligible, the specific

BAD ORIGINAL

0112299

- 5 -

antibodies are deluted with MgCl$_2$ 3.5 M and then dialyzed against water, concentrated under vacuum and dialyzed against PBS. The yield is of about 1.5 mg of specific antibody per ml of serum. It should be taken into consideration that by means of this method, an immunabsorbent can be used several times (>50) without any important loss of efficiency.

Thus the purification of the superoxidic-dismutasis purification takes place.

The first step is the preparation of the anti-superoxidic-dismutasis antibodies which are made insoluble on inert matrix. To this end, the specific antibodies are conjugated covalently on an inert matrix-in this case, Sepharose 4P (Trade Mark) by Pharmacia, activated with CNBr - and, as a consequence, about 90% of the antibodies will be covalently bound by the fixed phase.

Then the hemolysis product is prepared. For this purpose, one liter of cattle erythrocytes, washed with a 0.15 M solution of sodium chloride, is subjected to hemolysis with one liter of distilled water and left under cold agitation for 1 hour. Finally, the enzyme is purified with two liters of the hemolysis product passing on every 300 ml of immunoabsorbent which has been activated with specific anti-superoxidic-dismutasis antibodies. The column is washed with PBS.

Then, PBS added to sodium chloride 0.6 M is passed through and MgCl$_2$ 3.5 M is used to delute this latter. After dialysis against water the enzyme is concentrated

BAD ORIGINAL

with the solution passing on an anionic exchange resin column, - in this case GAL Sephadex (Trade Mark) by Pharmacia - buffered at pH 7.0 using a 5 mM sodium phosphate buffer. The dilution is then carried out with the same buffer 80 mM· 90 mg of superoxidic-dismutasis per 300 ml of immunoabsorbent are diluted on average. The superoxidic-dismutasis obtained in this way is desalinized and dehydrated. The paramagnetic resonance spectra (RPE) of the enzyme obtained in this way show the native protein well-known characteristics and the absence of any other paramagnetic species. The ultraviolet spectra, the electrophoresis in polyacrylamide and the specific activity of the enzyme show the absence of any contamination.

When hepatic tissue is used, this latter is finely sliced and homogenized by a mixing operation with an addition of a physiological solution.

The product of the invention can be used for pharmaceutical or industrial purposes.

CLAIMS

1. Method for superoxidic-dismutasis purification from animal tissues in particular, herythrocytes or hepatic tissue of superior animals, as Mammalia, Primates excepted, through chromatography by affinity using purified specific antibodies for the starting material, which have been suitably made insoluble on a proper matrix.

2. Method according to claim 1 and 2, characterized by the fact that specific antibodies are used which are produced either conventionally by using any animal species or by cellular hybrids (monoclonal antibodies of any species) technology.

3. Method according to claims 1, 2 characterized by the fact that the anti-superoxidic-dismutasie antibodies are purified by using the techniques of affinity on Staphylococcus protein A, made insoluble or of an antibody character, or anti-antibody made insoluble, or antigen made insoluble.

4. Method according to claim 1, wherein the starting material consists of erythrocytes, which are subjected to hemolysis.

5. Method according to claim 1, wherein the starting material consists of hepatic tissue, which is finely sliced, added to a physiologycal solution and then mixed up to obtain a homogenic mixture.

6. Superoxidic-dismutasis purified according to the method claimed in claims 1 to 5.